# EUROPEAN PATENT APPLICATION

(11) **EP 3 657 384 A1**
(43) Date of publication of application: **27.05.2020**
(21) Application number: 19207042.3
(22) Date of filing: 05.11.2019
(51) Int. Cl.: G06K 9/00, A61B 5/00

(54) **SKIN ANALYSIS APPARATUS**

(30) Priority: 22.11.2018 JP 2018219041
(71) Applicant: Panasonic Intellectual Property Management Co., Ltd., Osaka-shi, Osaka 540-6207 (JP)
(72) Inventor: TAOKA, Hiroki, Osaka-shi,, Osaka 540-6207 (JP); MATSUMOTO, Hiroshi, Osaka-shi,, Osaka 540-6207 (JP); TAKEI, Ichiro, Osaka-shi,, Osaka 540-6207 (JP)
(74) Representative: Grünecker Patent- und Rechtsanwälte PartG mbB

(57) **Abstract**

A skin analysis apparatus (10) includes: a housing (11); a camera (101) and a display (102) provided on a major surface of the housing; auxiliary mirrors, one of the auxiliary mirrors (22a) having a side end portion attached to a left-end portion of the housing, and the other auxiliary mirror (22b) having a side end portion being attached to a right-end portion of the housing; and a controller that causes the camera to capture images of a user's front-view and side-view face and that analyzes skin of the user's face by using the face images. An internal angle θ formed by a major surface of the display and a major surface of each auxiliary mirror is an angle at which the camera is capable of capturing an image of the user's side-view face while the user's front-view face is seen in the auxiliary mirror.

## Description

### BACKGROUND

### 1. Technical Field

The present disclosure relates to a skin analysis apparatus.

### 2. Description of the Related Art

Heretofore, the condition of the facial skin of a user has been analyzed based on face images captured by photographing the user's face from a plurality of different directions. Japanese Unexamined Patent Application Publication No. 2005-211581 discloses a face photographic apparatus in which a movable photography lighting unit having a camera and a lighting device rotates around a user's face to photograph the face from different directions.

### SUMMARY

An apparatus in which machine moves to photograph a user's face from different directions, as in Japanese Unexamined Patent Application Publication No. 2005-211581, tends to increase in its installation area, and for example, it is difficult to install the apparatus in a small space, such as a clinic.

Meanwhile, although an apparatus for photographing a user's face in different orientations by using a camera in front of the user as the user changes the orientation of his or her face makes it possible to reduce the installation area, it is difficult to reliably photograph the face in different orientations since the user's face is not fixable.

One non-limiting and exemplary embodiment provides a skin analysis apparatus that can reliably photograph a user's face in different orientations by using a camera in front of the user.

In one general aspect, the techniques disclosed here feature a skin analysis apparatus including: a housing; a camera and a display provided on a major surface of the housing; auxiliary mirrors, one of the auxiliary mirrors having a side end portion attached to a left-end portion of the housing, and the other auxiliary mirror having a side end portion being attached to a right-end portion of the housing; and a controller that causes the camera to capture images of a user's front-view and side-view face and that analyzes skin of the user's face by using the face images. An internal angle θ formed by a major surface of the display and a major surface of each auxiliary mirror is an angle at which the camera is capable of capturing an image of the user's side-view face while the user's front-view face is seen in the auxiliary mirror.

According to one aspect of the present disclosure, it is possible to reliably photograph a user's face in different orientations by using a camera in front of the user.

It should be noted that general or specific embodiments may be implemented as a system, a method, an integrated circuit, a computer program, or a recording medium or may be implemented by an arbitrary combination of a system, an apparatus, a method, an integrated circuit, a computer program, and a recording medium.

Additional benefits and advantages of the disclosed embodiments will become apparent from the specification and drawings. The benefits and/or advantages may be individually obtained by the various embodiments and features of the specification and drawings, which need not all be provided in order to obtain one or more of such benefits and/or advantages.

### BRIEF DESCRIPTION OF THE DRAWINGS

Fig. 1 is a schematic view illustrating an example of a use form of a skin analysis apparatus according to a first embodiment;
Fig. 2 is a flowchart illustrating an example of a use procedure of the skin analysis apparatus according to the first embodiment;
Fig. 3 is a diagram illustrating an example of a functional configuration of the skin analysis apparatus according to the first embodiment;
Fig. 4 is a view of an example in which the skin analysis apparatus according to the first embodiment is viewed from the front;
Fig. 5 is a view illustrating an example in which the skin analysis apparatus according to the first embodiment when auxiliary portions are opened is viewed from the top;
Fig. 6 is a view illustrating an example in which the skin analysis apparatus according to the first embodiment when the auxiliary portions are closed is viewed from the top;
Fig. 7 is a view illustrating an example of a photography guide user Interface (Ul) when an image of a front-view face is captured;
Fig. 8 is a view illustrating an example of the photography guide UI when an image of a right-side-view face is captured;
Fig. 9 is a view illustrating an example of the photography guide UI when an image of a left-side view face is captured;
Fig. 10 is a view illustrating an angle of view of acquisition of a face image;
Fig. 11 is a view illustrating an example in which a skin analysis apparatus according to a second embodiment is viewed from the front;
Fig. 12 is a view illustrating an example of a case in which auxiliary portions in the skin analysis apparatus according to the second embodiment are not accommodated;
Fig. 13 is a view illustrating an example of a case in which the auxiliary portions in the skin analysis apparatus according to the second embodiment are accommodated;
Fig. 14 is a view illustrating an example in which a skin analysis apparatus according to a third embodiment is viewed from the front;
Fig. 15 is a view illustrating a modification of the skin analysis apparatus according to the third embodiment; and
Fig. 16 is a diagram illustrating an example of a hardware configuration according to the present disclosure.

### DETAILED DESCRIPTION

Embodiments of the present disclosure will be described in detail with reference to the accompanying drawings, as appropriate. However, an overly detailed description may be omitted herein. For example, a detailed description of already well-known things and a redundant description of substantially the same configuration may be omitted herein. This is to avoid the following description becoming overly redundant and to facilitate understanding of those skilled in the art.

### (First Embodiment)

### <Example of Use of Skin Analysis Apparatus>

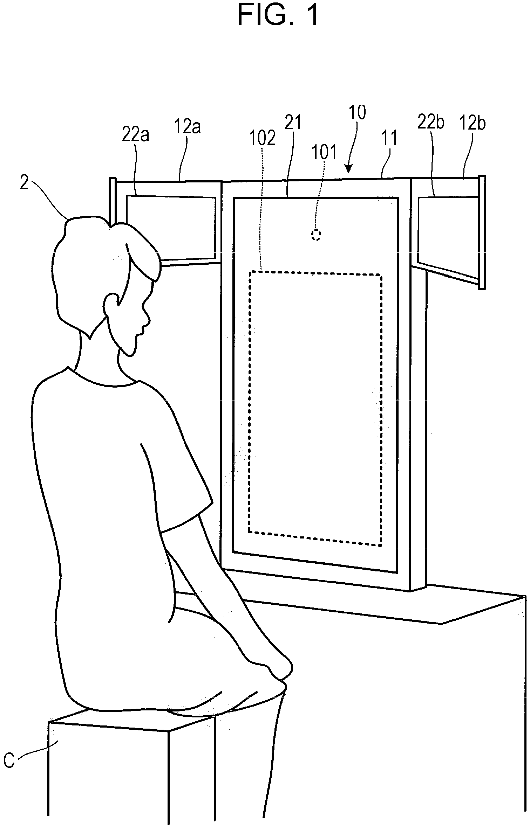

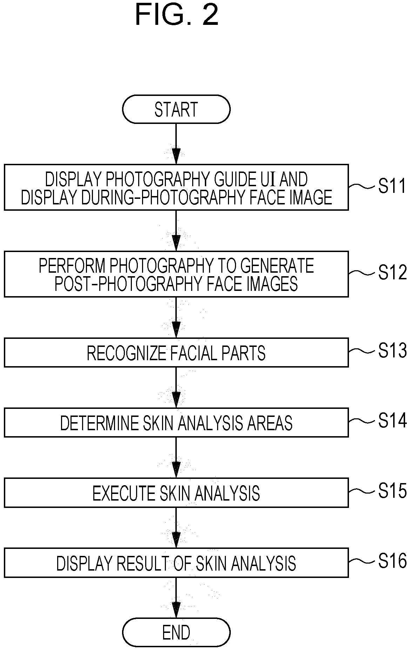

Fig. 1 illustrates an example of a use form of a skin analysis apparatus. Fig. 2 is a flowchart illustrating an example of a use procedure of the skin analysis apparatus. One example of a use procedure of the skin analysis apparatus will now be described with reference to Figs. 1 and 2.

A user 2 is seated in front of a skin analysis apparatus 10, as illustrated in Fig. 1, and gives a start instruction for starting skin analysis to the skin analysis apparatus 10. In response to the start instruction, the skin analysis apparatus 10 starts an operation below.

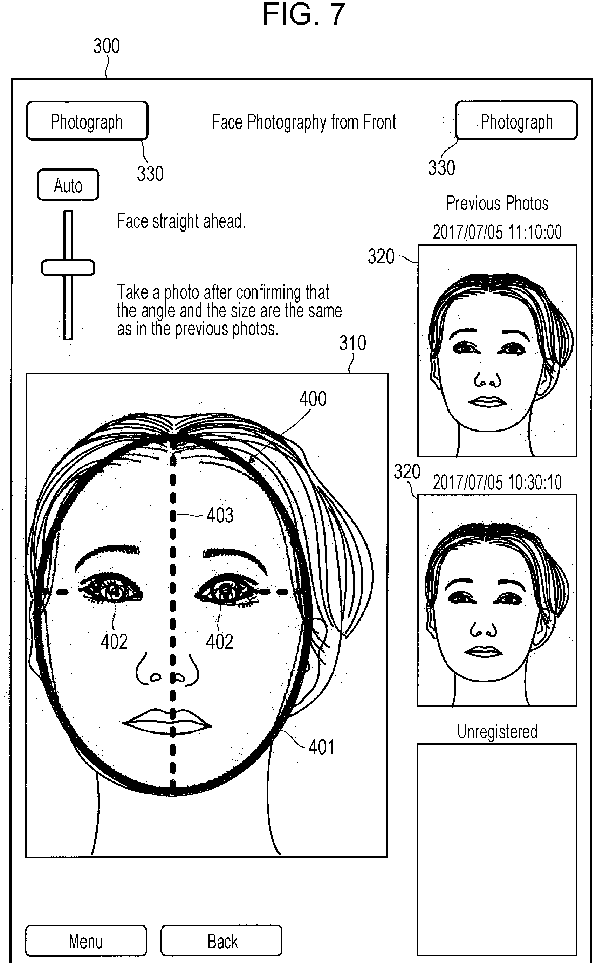

The skin analysis apparatus 10 displays, on a display 102, a photography guide user interface (Ul) 300 (see Figs. 7, 8, and 9) and a face image of the user 2 which is being captured by a camera 101 (this face image is hereinafter referred to as a "during-photography-face image") (S11). The photography guide UI 300 is a UI for giving guidance to the user 2 so that the face of the user 2 can be appropriately photographed. Details of the photography guide UI 300 are described later.

The skin analysis apparatus 10 instructs the user 2 so as to face straight ahead and captures a front-view face image of the user 2. The skin analysis apparatus 10 also instructs the user 2 to turn his or her face to the left and captures a right-side-view face image of the user 2. Also, the skin analysis apparatus 10 instructs the user 2 to turn his or her face to the right and captures a left-side-view face image of the user 2 (S12). The captured face images are referred to as "post-photography face images". Instead of instructing the user 2 so as to change the orientation of his or her face, the skin analysis apparatus 10 may automatically rotate a chair C in which the user 2 is seated to capture the right-side-view and left-side-view face images of the user 2.

The skin analysis apparatus 10 performs facial-part recognition processing on the post-photography face images (S13). Facial parts are characteristic parts in the face, and examples thereof include the contour of the face, the eyes, the nose, the mouth, the eyelids, and hairline. The facial parts may be represented as facial portions, facial organs, facial feature parts, or the like.

Based on the positions of the facial parts recognized in S13, the skin analysis apparatus 10 sets areas in which skin analysis is to be performed on the post-photography face images (the areas are hereinafter referred to as "skin analysis areas") (S14).

The skin analysis apparatus 10 executes the skin analysis on each skin analysis area set in S14 (S15).

The skin analysis apparatus 10 displays a result of the skin analysis, executed in S15, on the display 102 (S16).

By using the skin analysis apparatus 10, as described above, the user 2 can easily undergo the skin analysis. A detailed description will be given below.

### <Functional Configuration of Skin Analysis Apparatus>

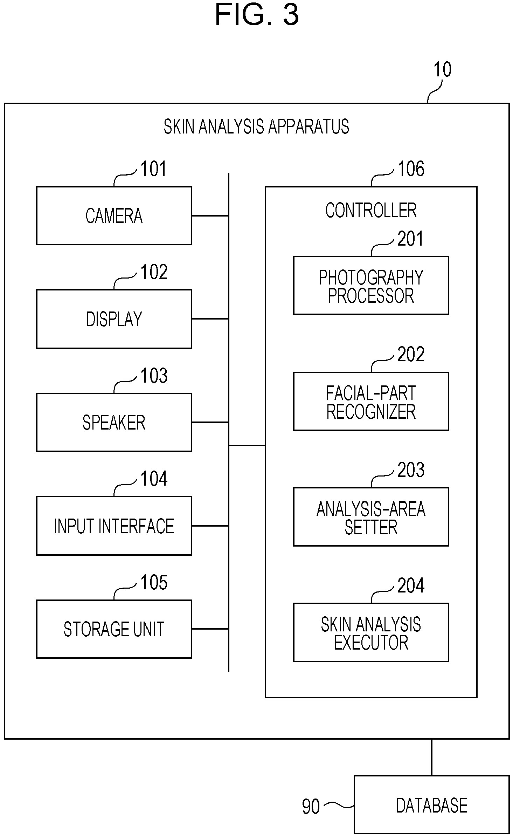

Next, a functional configuration of the skin analysis apparatus 10 will be described with reference to Fig. 3.

The skin analysis apparatus 10 includes, for example, the camera 101, the display 102, a speaker 103, an input interface 104, a storage unit 105, and a controller 106. The skin analysis apparatus 10 may also be connected to a database 90.

The camera 101 photographs the face of the user 2. Although the camera 101 is built into the skin analysis apparatus 10 in Fig. 1, the camera 101 may be a device independent from the skin analysis apparatus 10. In this case, the camera 101 transmits captured face images to the skin analysis apparatus 10 through a predetermined cable communication or wireless communication.

The display 102 displays images and information. Although the display 102 is built into the skin analysis apparatus 10 in Fig. 1, the display 102 may be a device independent from the skin analysis apparatus 10. In this case, the skin analysis apparatus 10 transmits data for display to the display 102 through a predetermined cable communication or wireless communication.

The speaker 103 outputs sound. For example, the speaker 103 outputs sound for notifying that the photography is started, the photography is ended, and so on.

The input interface 104 receives instructions from the user 2. The skin analysis apparatus 10 may include a plurality of input interfaces 104. For example, the skin analysis apparatus 10 may include a touch panel, a mouse, a keyboard, a button for photography instruction, and a microphone for voice input as the input interfaces 104. Each input interface 104 may also be a device independent from the skin analysis apparatus 10. In such a case, the input interface 104 transmits input data to the skin analysis apparatus 10 through a predetermined cable communication or wireless communication.

The storage unit 105 stores data used by the controller 106. The storage unit 105 may be a volatile memory, such as a dynamic random-access memory (DRAM), or a nonvolatile memory, such as a solid-state drive (SSD). Alternatively, the storage unit 105 may be a combination of a volatile memory and a nonvolatile memory.

The controller 106 is, for example, a central processing unit (CPU) and realizes functions of the skin analysis apparatus 10. For example, by executing a computer program stored in the storage unit 105, the controller 106 realizes functions associated with a photography processor 201, a facial-part recognizer 202, an analysis-area setter 203, and a skin analysis executor 204, which are described below.

The photography processor 201 generates the photography guide UI 300 and displays it on the display 102. The photography processor 201 also displays a during-photography-face image on the display 102 in real time. The photography processor 201 captures a front-view face image, a left-side-view face image, and a right-side-view face image of the user to generate post-photography face images.

For example, the facial-part recognizer 202 recognizes facial parts by performing the following processing. That is, first, the facial-part recognizer 202 extracts feature points from each face image by using a known image processing technique. Next, based on the extracted feature points, the facial-part recognizer 202 recognizes facial parts, such as the facial contour, eyes, nose, mouth, eyelid, and hairline. The facial-part recognizer 202 may perform facial-part recognition processing not only on the post-photography face images but also on face images during photography.

Based on the positions of the facial parts recognized by the facial-part recognizer 202, the analysis-area setter 203 sets at least one skin analysis area in any of the face images.

The skin analysis executor 204 executes skin analysis on each skin analysis area set in the face images by the analysis-area setter 203. For example, the skin analysis executor 204 applies known image processing to each skin analysis area in the face images to analyze, for example, the amounts of wrinkles, freckles, and/or pores.

Face images of users 2 and results of skin analysis on the face images are associated with each other and are managed in the database 90. Although the database 90 is a device independent from the skin analysis apparatus 10 in Fig. 3, the database 90 may be built into the skin analysis apparatus 10.

### <Physical Configuration of Skin Analysis Apparatus>

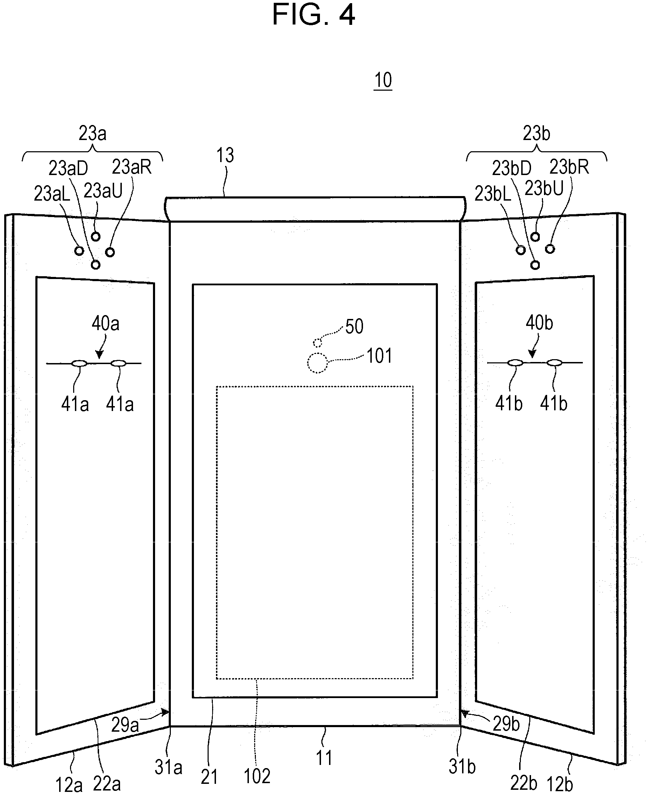

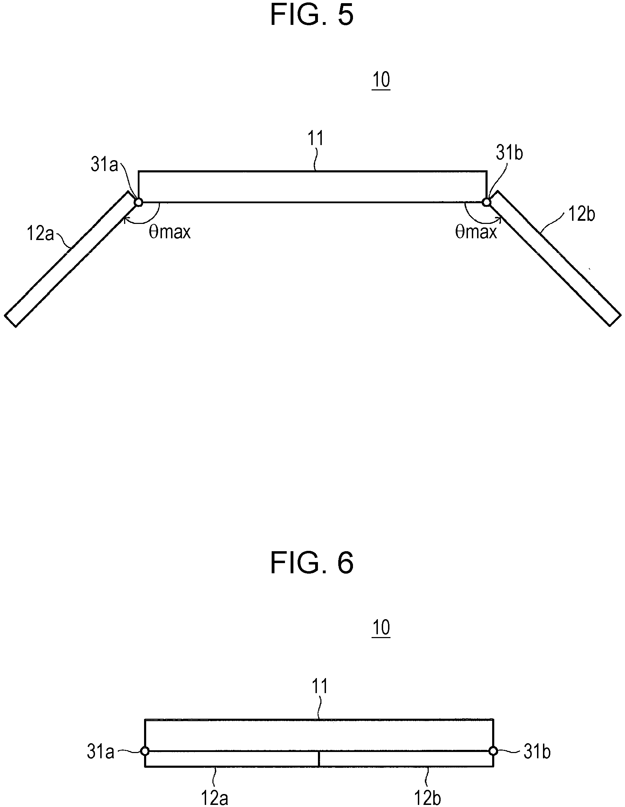

Next, a physical configuration of the skin analysis apparatus 10 according to a first embodiment will be described with reference to Figs. 4, 5, and 6. Fig. 4 illustrates one example in which the skin analysis apparatus 10 is viewed from the front. Fig. 5 illustrates one example in which the skin analysis apparatus 10 when a left auxiliary portion 12a and a right auxiliary portion 12b are opened is viewed from the top. Fig. 6 illustrates one example in which the skin analysis apparatus 10 when the left auxiliary portion 12a and the right auxiliary portion 12b are closed is viewed from the top. In Figs. 5 and 6, however, an upper light 13 is not illustrated.

As illustrated in Fig. 4, the skin analysis apparatus 10 includes a housing 11, the left auxiliary portion 12a, the right auxiliary portion 12b, the upper light 13, a mirror (front mirror) 21, the camera 101, and the display 102. When the left auxiliary portion 12a and the right auxiliary portion 12b are described without discrimination therebetween, they are referred to as "auxiliary portions 12".

The housing 11 has a flat-plate shape and has the front mirror 21, the camera 101, and the display 102 on its major surface facing the user 2. The display 102 is provided inside the front mirror 21. Alternatively, the display 102 may have a configuration integrated with the front mirror 21 (e.g., a mirror display having a half mirror). The camera 101 is provided above the display 102. The upper light 13 is provided at an upper end portion of the housing 11. The upper light 13 may be constituted by a plurality of light-emitting diode (LED) elements.

The left auxiliary portion 12a has a flat-plate shape and has, on its major surface facing the user 2, a mirror (left auxiliary mirror) 22a and direction instructions LED 23a, which are examples of light sources. Similarly, the right auxiliary portion 12b has a flat-plate shape and has, on its major surface facing the user 2, a mirror (right auxiliary mirror) 22b and direction instruction LEDs 23b, which are examples of light sources. Details of the direction instruction LEDs 23a and 23b are described later. When the direction instruction LEDs 23a on the left auxiliary portion 12a and the direction instruction LEDs 23b on the right auxiliary portion 12b are described without discrimination therebetween, they are referred to as "direction instruction LEDs 23".

As illustrated in Fig. 5, a right-side end portion 29a of the left auxiliary portion 12a is coupled to a left-end portion of the housing 11 via a hinge 31a. The left auxiliary portion 12a pivotally moves in a direction toward the front mirror 21 and in a direction away from the front mirror 21 about the axis of the hinge 31a. A left-side end portion 29b of the right auxiliary portion 12b is coupled to a right-end portion of the housing 11 via a hinge 31b. The right auxiliary portion 12b pivotally moves in a direction toward the front mirror 21 and in a direction away from the front mirror 21 about the axis of the hinge 31b.

As illustrated in Fig. 6, the left auxiliary portion 12a and the right auxiliary portion 12b may have sizes and shapes with which the front mirror 21 is covered when the left auxiliary portion 12a and the right auxiliary portion 12b are closed. That is, the front mirror 21, the left auxiliary mirror 22a, and the right auxiliary mirror 22b have a so-called three-sided mirror structure. Thus, when the skin analysis apparatus 10 is not used, the front mirror 21, the left auxiliary mirror 22a, and the right auxiliary mirror 22b are closed to thereby prevent exposure of the mirrors 21, 22a, and 22b. Hence, for example, when laser therapy is performed in a treatment room, it is possible to prevent a possibility that laser is reflected by the mirrors 21, 22a, and 22b.

The angle (internal angle) θmax formed by the major surface of the left auxiliary mirror 22a and the major surface of the front mirror 21 when the left auxiliary portion 12a is fully opened is an angle at which both eyes and the contour of the right-side-view face of the user 2 are appropriately captured by the camera 101 when the user 2 turns his or her face to the left, and the front-view face is seen in the left auxiliary mirror 22a. The hinge 31a has a lock mechanism for securing the left auxiliary portion 12a at the angle θmax. The right auxiliary portion 12b and the hinge 31b have structures that are the same as or similar to those of the left auxiliary portion 12a and the hinge 31a.

The left auxiliary portion 12a is provided with a marker 40a for adjusting the position and the size of the front-view face of the user 2 when the front-view face is seen in the left auxiliary mirror 22a. For example, the left auxiliary mirror 22a is provided with markers 41a for adjusting the positions of both eyes (these markers are hereinafter referred to as "eye markers"). The eye markers 41a may be provided at a height that is the same as the camera 101. The left auxiliary portion 12a may be provided with a marker for adjusting the position of the contour of the face (this marker is hereinafter referred to as a "contour marker", not illustrated), instead of or in addition to the eye markers 41a. Thus, when the user 2 turns to the left auxiliary portion 12a and adjusts the positions of both eyes and/or the contour of the face to the eye markers 41a and/or the contour marker, respectively, it is possible to reliably capture an image of the right-side-view face. The same also applies to eye markers 41b and a contour marker (not illustrated) on the right auxiliary portion 12b.

When the camera 101 is provided inside the front mirror 21, it is difficult for the user 2 to visually recognize the position of the camera 101. Accordingly, an LED 50, which is one example of a light source, is provided adjacent to the camera 101, and the controller 106 turns on the LED 50 during photography. This allows the user 2 to visually recognize the position of the camera 101 during photography and to direct his or her line-of-sight to the camera 101.

### <Photography Guide UI>

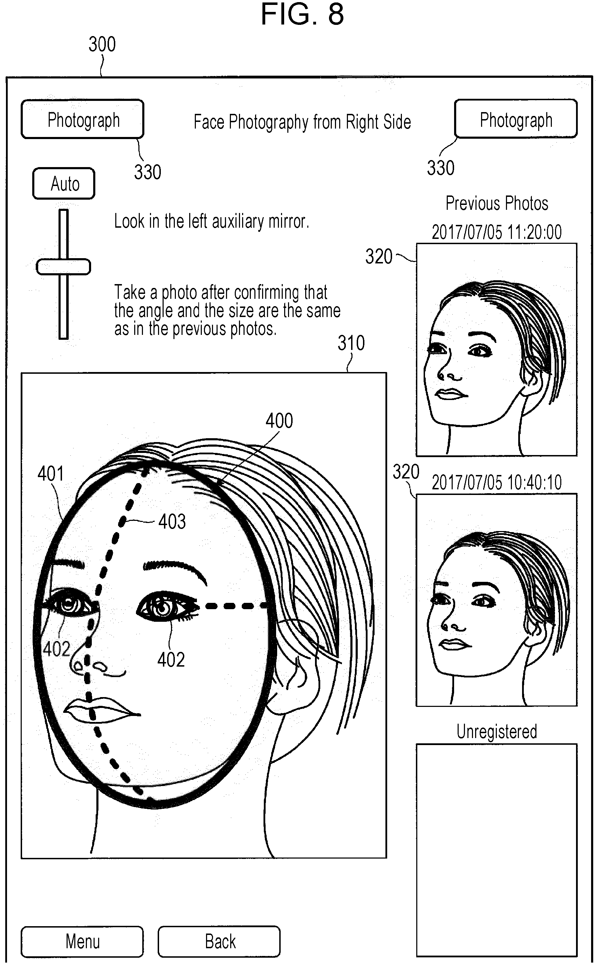

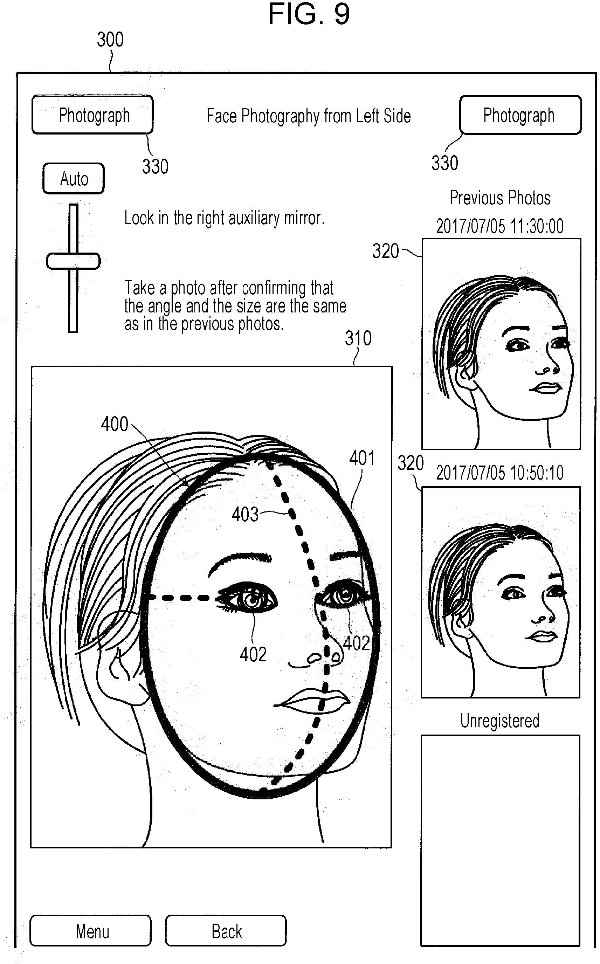

Next, the photography guide UI 300 will be described with reference to Figs. 7, 8, and 9. Fig. 7 is a view illustrating one example of the photography guide UI 300 when an image of the front-view face is captured. Fig. 8 is a view illustrating one example of the photography guide UI 300 when an image of the right-side-view face is captured. Fig. 9 is a view illustrating one example of the photography guide UI 300 when an image of the left-side-view face is captured.

The photography guide UI 300 has, for example, a during-photography-face image area 310, past-face-image areas 320, photograph buttons 330, and a face position guide 400.

A during-photography-face image of the user 2 is displayed in the during-photography-face image area 310. Face images of the same user 2 which were photographed in the past (these face images are hereinafter referred to as "past face images") are displayed in the past-face-image areas 320. The past face images are stored in the database 90.

For capturing an image of the front-view face, the photography processor 201 displays front-view past face images of the same user 2 in the past-face-image areas 320. Similarly, for capturing an image of the right-side-view face, the photography processor 201 displays right-side-view past face images of the same user 2 in the past-face-image areas 320, and for capturing an image of the left-side-view face, the photography processor 201 displays left-side-view past face images of the same user 2 in the past-face-image areas 320. Since the past face images are displayed together with the during-photography-face image, the user 2 can adjust the position, the size, and the orientation of the during-photography-face image so that they match the position, the size, and the orientation of the past face images by moving the position of the face. Thus, a skin analysis result of the past face images and a skin analysis result of the post-photography face images can be compared with each other with higher accuracy.

The photography processor 201 displays the face position guide 400 in the during-photography-face image area 310. The face position guide 400 includes a face contour line guide 401, eye position guides 402, and a face center line guide 403. The face contour line guide 401, the eye position guides 402, and the face center line guide 403 may have different arrangements depending on the orientation of the face to be photographed, as illustrated in Figs. 7, 8, and 9.

During photography of a face image, the user 2 adjusts the contour of the during-photography-face image to the face contour line guide 401, adjusts the eye positions in the during-photography-face image to the eye position guides 402, and adjusts the center line (the ridge of the nose) in the during-photography-face image to the face center line guide 403. This allows the photography processor 201 to capture a face image at an appropriate position, with an appropriate size, and in an appropriate orientation for performing skin analysis.

The photography processor 201 may use color of the face position guide 400 to indicate whether or not the during-photography-face image matches the position, the size, and the orientation of the face position guide 400. For example, when the facial-part recognizer 202 succeeds in recognizing facial parts, and the during-photography-face image matches the position, the size, and the orientation of the face position guide 400, the photography processor 201 may switch the color of the face position guide 400 to blue. When the facial-part recognizer 202 succeeds in recognizing facial parts, and the during-photography-face image does not match the position, the size, and the orientation of the face position guide 400, the photography processor 201 may switch the color of the face position guide 400 to red. Also, when the facial-part recognizer 202 fails in recognizing facial parts, the photography processor 201 may switch the color of the face position guide 400 to orange. When the during-photography-face image does not match the position, the size, and the orientation of the face position guide 400, the photography processor 201 does not have to start photography. This makes it possible to efficiently capture an appropriate face image for skin analysis.

The photograph buttons 330 are respectively provided at a left-end portion and a right-end portion in the photography guide UI 300. Thus, at whichever of the left and right sides of the skin analysis apparatus 10 health personnel (e.g., a nurse) or the like is situated, the health personnel can touch the photograph button 330 without crossing between the camera 101 and the display 102 and the face of the user 2. The photograph button 330 may be provided at one of the left-end portion and the right-end portion in the photography guide UI 300, and the position of the photograph button 330 may be switchable through setting.

### <Direction Instruction LEDs>

While the user 2 is facing one of the auxiliary mirrors 22 (i.e., during photography of the side view of the face), he or she cannot see the face position guide 400 displayed on the display 102 in front of the user 2. When the user 2 is looking in the auxiliary mirror 22, the direction instruction LEDs 23 on each auxiliary portion 12 are used in order to give guidance for the orientation of the face to the user 2 so that a during-photography-face image of the side view of the face matches the face position guide 400.

Next, a description will be given of one example of the operation of the direction instruction LEDs 23a provided on the left auxiliary portion 12a. The same also applies to the operation of the direction instruction LEDs 23b provided on the right auxiliary portion 12b.

For example, when a during-photography-face image of the right view of the face is facing upward too much, the photography processor 201 turns on (or blinks) a direction instruction LED 23aD indicating "down". That is, the user 2 is instructed so as to face downward a little. When a during-photography-face image of the right-side view of the face is facing downward too much, the photography processor 201 turns on (or blinks) a direction instruction LED 23aU indicating "up". That is, the photography processor 201 instructs the user 2 so as to face upward a little. When a during-photography-face image of the right-side view of the face is facing leftward too much, the photography processor 201 turns on (or blinks) a direction instruction LED 23aR indicating "right". That is, the photography processor 201 instructs the user 2 so as to face rightward a little. When a during-photography-face image of the right-side view of the face is facing rightward too much, the photography processor 201 turns on (or blinks) a direction instruction LED 23aL indicating "left". That is, the photography processor 201 instructs the user 2 so as to face leftward a little. When a during-photography-face image of the right-side view of the face is in an appropriate orientation, the photography processor 201 turns on (blinks) all the direction instruction LEDs 23aD, 23aU, 23aR, and 23aL. That is, the photography processor 201 notifies the user 2 that the during-photography-face image is in a correct orientation.

### <When Photography Is Started and When Photography Is Completed>

Next, one example of the operation of the photography processor 201 when photography is started and when photography is completed will be described in detail.

Immediately before the right-side view of the face is photographed, and immediately before the left-side view of the face is photographed, the photography processor 201 may cause the speaker 103 to output sound indicating that the photography is started. This allows the photography processor 201 to give a notification indicating the start of the photography to the user 2 who is facing left or right and having difficulty in seeing the photography guide UI 300 displayed on the display 102 in front of the user 2. After the photography is completed, the photography processor 201 may also cause the speaker 103 to output sound indicating that the photography is completed.

Also, by using the direction instruction LEDs 23 provided on each auxiliary portion 12, the photography processor 201 may give notifications indicating that the photography is started and the photography is completed. For example, the photography processor 201 may blink all the direction instruction LEDs 23 immediately before the photography is started and may turn off all the direction instruction LEDs 23 after the photography is completed.

The facial-part recognizer 202 determines whether or not the hair covers the area of the forehead in the during-photography-face image. Upon determining that the hair covers the area of the forehead, the facial-part recognizer 202 may display, on the photography guide UI 300, an instruction for fixing the hair. In this case, the photography processor 201 does not have to start the photography.

For photographing the front-view face, the photography processor 201 may adjust the focus of the camera 101 to the positions of both eyes. For photographing the right-side view face, the photography processor 201 may adjust the focus of the camera 101 to the position of the right eye (the eye closer to the camera 101). For photographing the left-side view face, the photography processor 201 may adjust the focus of the camera 101 to the position of the left eye (the eye closer to the camera 101). Adjusting the focus of the camera 101 in such a manner makes it possible to capture a face image that is appropriate for skin analysis, since the eyes are located in the vicinity of the center in the depth direction of the face.

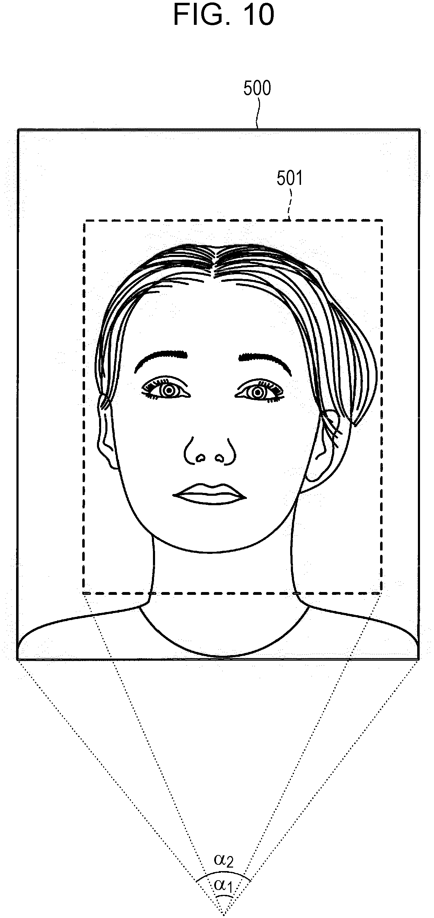

As illustrated in Fig. 10, the photography processor 201 may perform photography with an angle of view α₂, which is larger than an angle of view α₁ including only the face. Then, the photography processor 201 may generate a post-photography face image by extracting, from an image 500 captured with the angle of view α₂, an area 501 in which a face image is captured. Thus, for example, after photography, the photography processor 201 can adjust the post-photography face image so that it matches the past face images.

Also, for capturing a plurality of face images in the same orientation, the photography processor 201 may correct post-photography face images so that the sizes and positions of the face match each other in the post-photography face images. For example, the photography processor 201 captures a first face image with the upper light 13 illuminating the face with horizontally polarized light, captures a second face image with the upper light 13 illuminating the face in the same orientation with vertically polarized light, and captures a third face image without illumination. When the sizes and/or the positions of the face are displaced in the first, second, and third face images, the photography processor 201 corrects the first, second, and third face images so that the sizes and/or the positions of the face match each other. The photography processor 201 may detect displacements among the post-photography images by using a known template matching technique.

### (Second Embodiment)

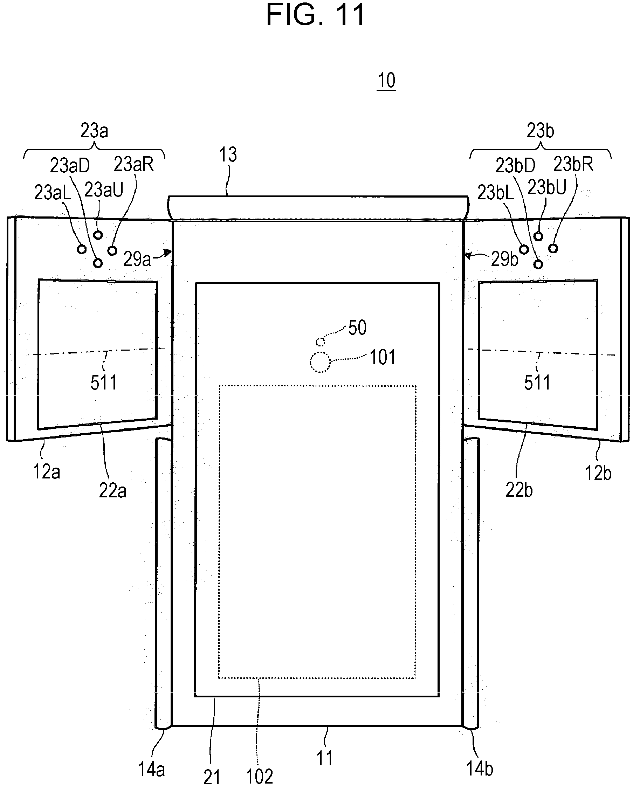

A physical configuration example of a skin analysis apparatus 10 according to a second embodiment will be described with reference to Fig. 11. A main difference in Fig. 11 from Fig. 4 is that the sizes of auxiliary portions 12 are small. Descriptions of constituent elements that are the same as or similar to those in the first embodiment may be omitted hereinafter.

As illustrated in Fig. 11, the skin analysis apparatus 10 includes a housing 11, a left auxiliary portion 12a, a right auxiliary portion 12b, an upper light 13, a left light 14a, a right light 14b, a front mirror 21, a camera 101, and a display 102. Since the housing 11, the upper light 13, the front mirror 21, the camera 101, and the display 102 are the same as or similar to those illustrated in Fig. 4, descriptions thereof are not given hereinafter.

A right-side end portion 29a of the left auxiliary portion 12a is attached to a position of a left-end portion of the housing 11, the position being located where the height of the camera 101 is between an upper end and a lower end of a left auxiliary mirror 22a. Similarly, the left-side end portion 29b of the right auxiliary portion 12b is attached to a position of a right-end portion of the housing 11, the position being located where the height of the camera 101 is between an upper end and a lower end of the right auxiliary mirror 22b. Each of the left auxiliary portion 12a and the right auxiliary portion 12b is attached to the housing 11 with the angle θmax described above with reference to Fig. 5.

The size of the left auxiliary mirror 22a is a size into which the entire front-view face seen in the left auxiliary mirror 22a generally fits when the user 2 turns his or her face to the left. The same applies to the size of the right auxiliary mirror 22b. In other words, in Fig. 11, at least one of the left and right auxiliary mirrors 22 may be larger than the entire front-view face of the user 2 which is seen in the auxiliary mirror 22 when the user 2 turns his or her face away from the camera 101, and the vertical and horizontal dimensions of the auxiliary mirror 22 may be respectively smaller than the vertical and horizontal dimensions of the front mirror 21.

The left light 14a is provided at a portion that is included in the left-end portion of the housing 11 and that is located below the left auxiliary portion 12a. The right light 14b is provided at a portion that is included in the right-end portion of the housing 11 and that is located below the right auxiliary portion 12b.

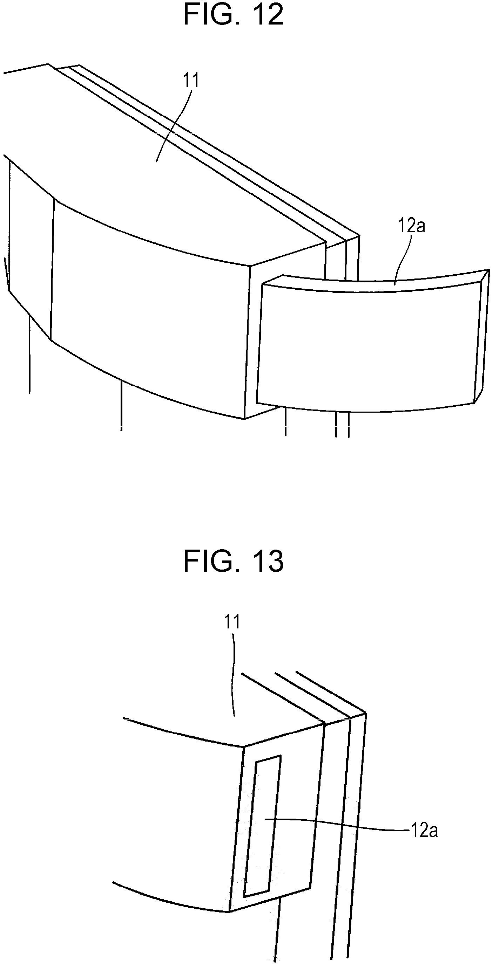

As illustrated in Figs. 12 and 13, the auxiliary portions 12 may be able to be accommodated in the housing 11 via slide mechanisms. This allows the auxiliary portions 12 to be accommodated in the housing 11 when the skin analysis apparatus 10 is not used and makes it possible to prevent health personnel, an examinee, or the like in a treatment room from inadvertently hitting the auxiliary portions 12.

As illustrated in Fig. 11, the auxiliary mirrors 22 may be adjustable in their angles φ about a horizontal axis 511. This allows the surfaces of the auxiliary mirrors 22 to be adjusted to the height position of the face of the user 2.

### (Third Embodiment)

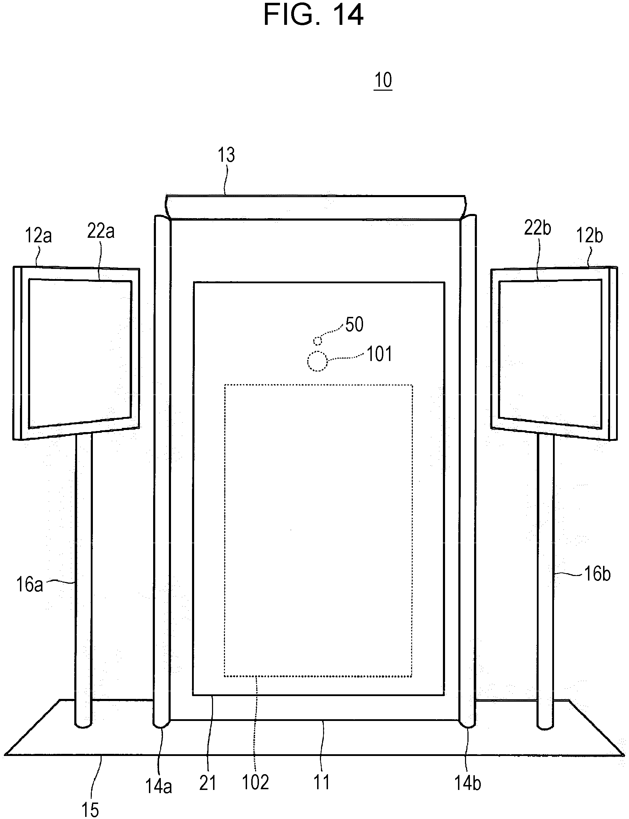

A physical configuration example of a skin analysis apparatus 10 according to a third embodiment will be described with reference to Fig. 14. A main difference in Fig. 14 from Fig. 11 is that auxiliary portions 12 are separated from a housing 11. Descriptions of constituent elements that are same as or similar to those in the second embodiment may be omitted hereinafter.

As illustrated in Fig. 14, the skin analysis apparatus 10 includes the housing 11, a left auxiliary portion 12a, a right auxiliary portion 12b, an upper light 13, a left light 14a, a right light 14b, a pedestal 15, a left support 16a, a right support 16b, a front mirror 21, a camera 101, and a display 102.

The housing 11 is secured to the pedestal 15. Since the housing 11, the upper light 13, the front mirror 21, the camera 101, and the display 102 are the same as or similar to those in Fig. 4, descriptions thereof are not given hereinafter.

The left support 16a is secured to a position that is included in the pedestal 15 and that is located at the left side of the housing 11, and extends in a height direction. The left auxiliary portion 12a is secured to an upper end portion of the left support 16a so that the left auxiliary portion 12a and the front mirror 21 form the angle θmax, which is described above with reference to Fig. 5. The right support 16b is secured to a position that is included in the pedestal 15 and that is located at the right side of the housing 11, and extends in the height direction. The right auxiliary portion 12b is secured to an upper end portion of the right support 16b so that the right auxiliary portion 12b and the front mirror 21 form the angle θmax, which is described above with reference to Fig. 5.

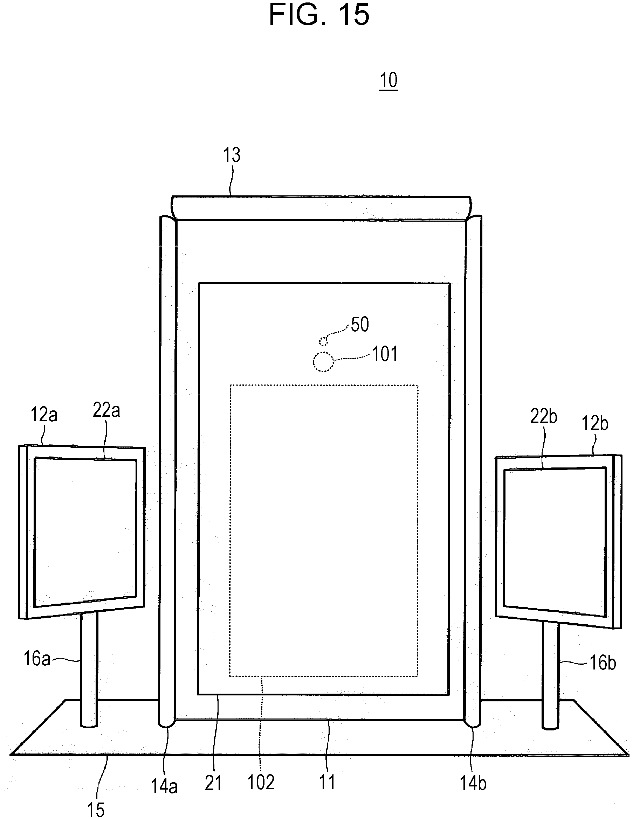

The left auxiliary portion 12a may also be secured to the left support 16a so that the height of the camera 101 is located between an upper end and a lower end of the left auxiliary mirror 22a. Similarly, the right auxiliary portion 12b may be secured to the right support 16b so that the height of the camera 101 is located between an upper end and a lower end of the right auxiliary mirror 22b. However, the heights of the left auxiliary portion 12a and the right auxiliary portion 12b are not limited to those heights and may be, for example, smaller than the height of the camera 101, as illustrated in Fig. 15.

The left light 14a and the right light 14b are respectively provided at the left-end portion and the right-end portion of the housing 11. Compared with the structure illustrated in Fig. 11, the auxiliary portions 12 are separated from the housing 11, as described above, and thus the left light 14a and the right light 14b that are vertically longer than those in Fig. 11 can be respectively provided at the left-end portion and the right-end portion of the housing 11.

### <Brief Summary of the Present Disclosure>

A skin analysis apparatus 10 according to the present disclosure includes a housing 11, a camera 101 and a display 102 provided on a major surface of the housing 11, auxiliary portions 12a and 12b having side end portions 29a and 29b attached to respective left-end and right-end portion of the housing 11, and a controller 106 that causes the camera 101 to capture images of the front-view and side-view face of a user 2 and that analyzes skin of the face of the user 2 by using the images. An internal angle θ formed by a major surface of the display 102 and a major surface of each of the auxiliary portions 12a and 12b is an angle at which the camera 101 is capable of capturing an image of the side-view face of the user 2 while the front-view face of the user 2 is seen in the corresponding auxiliary portion 12a or 12b. In other words, for example, the internal angle θ may be an angle at which the camera 101 is capable of photographing at least both eyes of the user 2 (i.e., is capable of seeing both eyes). Alternatively, the internal angle θ may be an angle at which the camera 101 is capable of photographing the contour of a near side and a far side of the face of the user 2 relative to the camera 101. Alternatively, the internal angle θ may be an angle at which the contour of the cheek at a far side of the face of the user 2 relative to the camera 101 is not hidden by the nose. Alternatively, the internal angle θ may be an angle at which the camera 101 is capable of photographing both eyes of the user 2 and a contour of the face of the user 2 from the ear at a near side of the user 2 relative to the camera 101 to the chin of the user 2.

According to the configuration, when the user 2 turns his or her face to one of the auxiliary mirrors 22a and 22b so that his or her front-view face is seen in the auxiliary mirror 22a or 22b, the camera 101 in front of the user 2 can capture an image of his or her side-view face reliably (i.e., in substantially the same orientation). Thus, a skin analysis result of a face image captured in the current photography and a skin analysis result of a face image captured in past photography can be compared with each other with higher accuracy.

The embodiments according to the present disclosure have been described above in detail with reference to the accompanying drawings, and the functions of the skin analysis apparatus 10 can be realized by a computer program.

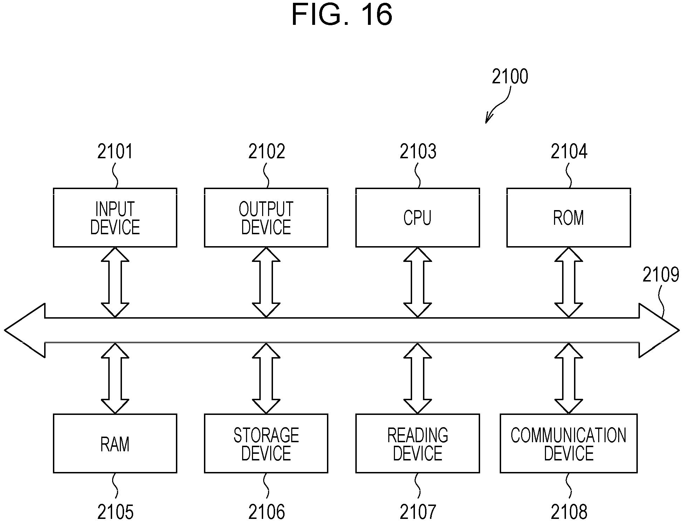

Fig. 16 is a diagram illustrating a hardware configuration of a computer that realizes the functions of each apparatus by using a program. A computer 2100 includes an input device 2101, such as a keyboard, a mouse, or a touchpad, an output device 2102, such as the display 102 or a speaker, a CPU 2103, a reading device 2107 that reads information from a storage device 2106, such as a read-only memory (ROM) 2104, a random-access memory (RAM) 2105, a hard-disk device, or an SSD, or a recording medium, such as a digital versatile disk read-only memory (DVD-ROM) or a Universal Serial Bus (USB) memory, and a communication device 2108 that performs communication through a network. These individual elements are connected through a bus 2109.

The reading device 2107 reads a program for realizing the functions of each apparatus described above from a recording medium on which the program is recorded and causes the program to be stored in the storage device 2106. Alternatively, the communication device 2108 communicates with a server apparatus, connected to a network, to download a program for realizing the functions of each apparatus from the server apparatus and causes the downloaded program to be stored in the storage device 2106.

The CPU 2103 copies the program, stored in the storage device 2106, to the RAM 2105, sequentially reads instructions included in the program from the RAM 2105, and executes the instructions to thereby realize the functions of each apparatus.

The present disclosure can be realized by software, hardware, or software that cooperates with hardware.

Each functional block used in the description of each embodiment above can be partly or entirely realized by an LSI, which is an integrated circuit, and each process described in each embodiment above may be controlled partly or entirely by one LSI or a combination of LSIs. The LSI may be individually formed as chips or may be formed by one chip so as to include a part or all of the functional blocks. The LSI may include an input and an output of data. The LSI may be referred to as an IC, a system LSI, a super LSI, or an ultra LSI depending on a difference in the degree of integration.

The technique of the circuit integration is not limited to the LSI and may be realized by using a dedicated circuit, a general-purpose processor, or a special-purpose processor. Also, a field programmable gate array (FPGA) that can be programmed after the manufacture of the LSI or a reconfigurable processor in which the connections and the settings of circuit cells arranged inside the LSI can be reconfigured may be used.

In addition, when a technology for circuit integration that replaces LSI becomes available with the advancement of semiconductor technology or another derivative technology, such a technology may also naturally be used to integrate the functional blocks. Application of biotechnology or the like is possible.

One aspect of the present disclosure is useful for a system that photographs the face.

## Claims

1. A skin analysis apparatus comprising:
a housing;
a camera and a display provided on a major surface of the housing;
auxiliary mirrors, one of the auxiliary mirrors having a side end portion attached to a left-end portion of the housing, and the other auxiliary mirror having a side end portion being attached to a right-end portion of the housing; and
a controller that causes the camera to capture images of a user's front-view and side-view face and that analyzes skin of the user's face by using the face images,
wherein an internal angle θ formed by a major surface of the display and a major surface of each auxiliary mirror is an angle at which the camera is capable of capturing an image of the user's side-view face while the user's front-view face is seen in the auxiliary mirror.

2. The skin analysis apparatus according to Claim 1,
wherein the angle at which the camera is capable of capturing the image of the user's side-view face is an angle at which the camera is capable of photographing the user's both eyes.

3. The skin analysis apparatus according to Claim 1,
wherein the angle at which the camera is capable of capturing the image of the user's side-view face is an angle at which the camera is capable of photographing a contour of both a near side and a far side of the user's face relative to the camera.

4. The skin analysis apparatus according to Claim 1,
wherein the angle at which the camera is capable of capturing the image of the user's side-view face is an angle at which the camera is capable of photographing both the user's both eyes and a contour of the user's face from the user's ear at a near side relative to the camera to the user's chin.

5. The skin analysis apparatus according to Claim 1,
wherein each auxiliary mirror is provided with a marker for adjusting positions of eyes while the front-view face is seen in the auxiliary mirror.

6. The skin analysis apparatus according to Claim 1,
wherein each auxiliary mirror is coupled to the housing via a hinge and pivotally moves about an axis of the hinge in a direction in which the internal angle θ decreases.

7. The skin analysis apparatus according to Claim 1,
wherein each auxiliary mirror has a size into which the user's entire front-view face seen in the auxiliary mirror fits when the user turns his or her face away from the camera.

8. The skin analysis apparatus according to Claim 7,
wherein a height of the camera is located between an upper end and a lower end of each auxiliary mirror.

9. The skin analysis apparatus according to Claim 7,
wherein each auxiliary mirror pivotally moves in a direction in which the auxiliary mirror is accommodated in the housing.

10. The skin analysis apparatus according to Claim 1,
wherein a first light is provided adjacent to the camera, and
the controller turns on the first light during acquisition of an image of the front-view face.

11. The skin analysis apparatus according to Claim 1,
wherein one or more second lights are provided adjacent to each auxiliary mirror, and
the controller turns on any of the one or more second lights during acquisition of an image of the side-view face.

12. The skin analysis apparatus according to Claim 11,
wherein the one or more second lights are arranged to indicate up-and-down directions and/or left-and-right directions, and
when an orientation of the image of the side-view face is displaced from a predetermined face orientation, the controller turns on the second light corresponding to a direction in which the displacement is to be corrected.

13. The skin analysis apparatus according to Claim 1,
wherein, for capturing an image of the front-view face, the controller adjusts a focus of the camera to the user's both eyes, and for capturing an image of the side-view face, the controller adjusts the focus of the camera to the user's eye closer to the camera.

14. The skin analysis apparatus according to Claim 1,
wherein, for capturing an image of the front-view face, the controller causes a front-view face guide for adjusting a position and a size of the front-view face to be displayed on the display, and for capturing an image of the side-view face, the controller causes a side-view face guide for adjusting a position and a size of the side-view face to be displayed on the display.

15. The skin analysis apparatus according to Claim 14,
wherein, when a position of a face image displayed on the display does not match the position and the size of the corresponding guide, the controller does not start capturing the face image.

16. The skin analysis apparatus according to Claim 14,
wherein, depending on whether or not a position of a face image displayed on the display matches the position and the size of the corresponding guide, the controller switches a mode of the guide.

17. The skin analysis apparatus according to Claim 1,
wherein, upon detecting that the user's hair in a face image displayed on the display covers an area of the user's forehead therein, the controller does not start capturing the face image.

18. The skin analysis apparatus according to Claim 1,
wherein the controller causes a photograph button for receiving start of the photography to be displayed at each of a left-end portion and a right-end portion of the display.

19. The skin analysis apparatus according to Claim 1,
wherein the controller causes the user's previously captured face image to be displayed on the display in conjunction with the user's face image that is being captured.
